# EUROPEAN PATENT APPLICATION

(11) **EP 1 673 985 A1**
(43) Date of publication of application: **28.06.2006**
(21) Application number: 04788010.9
(22) Date of filing: 22.09.2004
(51) Int. Cl.: A23L 1/22, A23L 1/30, A61K 31/19, A61K 31/70, A61P 3/10

(54) **FUNCTIONAL SWEETENER**

(30) Priority: 22.09.2003 US 503891 P
(71) Applicant: Use-Techno Corporation, Fukuchiyama-shi, Kyoto 6200055 (JP)
(72) Inventor: MATSUYAMA, Futoshi, 6208502 (JP)
(74) Representative: Ouzman, Beverley Nicola Claire
(86) International application number: PCT/JP2004/013827
(87) International publication number: WO 2005/027656

(57) **Abstract**

The present invention provides a sweetener wherein corosolic acid, and at least one selected from the group consisiting of a sucrase inhibitor and indigestible dextrin, are added to sucrose.

## Description

### Technical Field

The present invention relates to a functional sweetener.

### Background Art

Corosolic acid is present in many types of plants, but is now mainly obtained from banaba *(Lagerstroemia speciosa (L.) Pers.).* Banaba belongs to *Myrtales Lythraceae,* and is a kind of crape myrtle distributed throughout tropical Asia, which is known as Pride of India. A hot water decoction of this leaf has been taken as a therapeutic agent in the Philippines and some other countries for many years. Recently, it has been attracting interest in Japan for its diabetes treating effect and blood glucose level depressing effect, and the number of people who take it as a healthy tea is now increasing.

Regarding the medicinal value of banaba, it was already reported in the 1940s that when a healthy domestic rabbit was medicated with a decoction of desiccated banaba leaves, blood glucose level decreased by 16-49 mg/dl with a dose of 1-2 g of desiccated leaves per 1 kg of body weight (Non-Patent Document 1). Further, in Patent Document 1, it is reported that type II diabetic mice which had been fed with a diet containing 3% mixed banaba extract powder for one week, and thereafter fed with a diet containing 5% mixed banaba extract powder for 3 weeks, significantly lowered the blood glucose levels compared with control mice which had not received the banaba extract powder.
Patent Document 1: Japanese Patent Application Laid-Open No. 5-310587
Non-Patent Document 1: F. Garcia, J. Philip. Med. Assoc., 20, 395 (1940)

### Disclosure of the Invention

### Problem to be Solved by the Invention

In Japanese Patent Application Laid-Open No. 9-227398, it is reported that this medicinal effect of banaba is partly due to the inhibition of amylase and lipase, and in Japanese Patent Application Laid-Open No. 2002-12547, it was observed that as a result of this inhibitory effect, blood glucose elevation was suppressed when saccharides were simultaneously ingested. However, according to the inventor's research, the blood glucose level depressing effect of banaba extract is mainly due to the action of corosolic acid, and the contribution of digestive enzyme inhibitors is secondary.

According to the inventor's latest research, the blood glucose level depressing mechanism of corosolic acid is as follows. Specifically, corosolic acid stimulates the early-phase secretion of insulin. The insulin secretion stimulated by corosolic acid then stimulates the uptake by peripheral cells of glucose which has started to increase in the blood, and it results in decrease of the blood glucose level. The reduction of the blood glucose level inhibits the elevation of insulin secretion which would usually have been a result of the increase in glucose, and as a result, the corosolic acid controls the total amount of insulin secretion. Corosolic acid also has an insulin-like effect of promoting glucose uptake by peripheral cells, as has been pointed out in the past, and will therefore suppress blood glucose also from this viewpoint. Moreover, corosolic acid only lowers the blood glucose level in the presence of blood glucose of a certain concentration or higher, so corosolic acid is an excellent food additive, and is also a safer and more effective antidiabetic substance than ordinary antidiabetic drugs.

While it is important to control the blood glucose level in treatment of diabetes, it is recently also said to be important to suppress excessive insulin secretion because it affects overall body health, and many low insulin food products are now being marketed in the U.S. Although corosolic acid initially temporarily stimulates insulin secretion, it suppresses total insulin secretion, and is therefore of importance as a low insulin substance. Further, as rapid fluctuations of blood glucose affect the nervous system, it is necessary to maintain insulin stable at a low level. Corosolic acid is a substance which meets this demand perfectly.

However, supposing that blood glucose level depression by corosolic acid follows the above mechanism, blood glucose rapidly increases before insulin secretion is stimulated by corosolic acid, if corosolic acid and sugar which is rapidly absorbed are consumed together.

In this case also, glucose uptake by peripheral cells is still promoted by corosolic acid, so the blood glucose level stabilizes more quickly than if corosolic acid was not consumed, but if its effect of stimulating the early-phase secretion of insulin could also be utilized, an even more pronounced blood glucose level depressing effect of corosolic acid might be obtained.

### Means for Solving the Problem

The inventor focused on the contribution of digestive enzyme inhibitors present in banaba extract which was a secondary effect found from prior research, and thought as follows. The reason why a remarkable blood glucose level depressing effect is obtained by consuming banaba extract is not because digestive enzyme inhibitors interfere with the digestion and absorption of sugar, but because the digestion and absorption of sugar is delayed. During this time, the absorbed corosolic acid stimulates insulin secretion, so the uptake by peripheral cells of the sugar with slightly delayed absorption is promoted. This thought was also supported by the fact that, in *in vivo* experiments on rats, if banaba extract was given together with starch, which is a polysaccharide, the blood glucose level was depressed, but if it was given together with glucose, which is a monosaccharide, the blood glucose level was not depressed (Japanese Patent Application Laid-Open No. 2002-12547). On the basis of the above thought, the inventor concluded that the early insulin secretion stimulating effect of colosolic acid could be utilized and a blood glucose level depressing effect identical to that of banaba extract might be obtained, by combining corosolic acid with known digestive enzyme inhibitors or digestion and absorption inhibitors, and that by such a combination, the disadvantages of tannin and the like contained in the extract could be avoided.

The present invention provides a sweetener (functional sweetener) wherein corosolic acid, and at least one selected from the group consisting of a sucrase inhibitor and indigestible dextrin, are added to sucrose. The sucrase inhibitor is preferably L-arabinose or 1-deoxynojirimycin. Indigestible dextrin is a substance which suppresses rapid elevation of blood glucose level by interfering with the digestion and absorption of sugars.

The sweetener of the present invention may contain other additive components to the extent that they do not interfere with its effect.

### Effects of the Invention

According to the sweetener (functional sweetener or functional sugar) of the present invention, compared to the case where sucrose is consumed alone, or the case where sucrose is consumed together with a sucrase inhibitor or a digestion and absorption inhibitor (blood glucose level depressor), blood glucose level is stabilized. This sweetener has a blood glucose level depressing action identical to that of banaba extract, and does not contain tannin, nor other components having undesirable actions, which are present in banaba extract.

### Best Modes for Carrying Out the Invention

### (Isolation and purification of corosolic acid)

The corosolic acid contained in the sweetener (functional sweetener) of the present invention may be manufactured from banaba extract or banaba extract concentrate.

Banaba extract is obtained by extracting banaba leaves with hot water, an alcohol such as methanol, ethanol or propanol, or an aqueous solution of these alcohols. It contains corosolic acid and banaba polyphenols (polyphenols in banaba leaves), which are the principal components, and the extraction can be performed by the following method.

Banaba leaves used as the raw material for banaba extract are the fresh or dried leaves of Banaba (*Lagerstroemia speciosa Linn.* or *Pers*.), which grows for example in the Philippines. The fresh leaves may be dried for example by natural drying, air drying or forced drying. The drying is preferably performed to a "toasted dry" state with a moisture content of no greater than 20 wt% and preferably no greater than 10 wt% in order to prevent growth of microorganisms and ensure storage stability.

The dried banaba leaves may be extracted directly, but they may preferably be extracted after pulverization and chopping. The methods and conditions employed to extract the dried banaba leaves with hot water or alcohol and to concentrate the extract are not particularly limited, but they are preferably such as to yield a fixed content of corosolic acid in the concentrate. Specifically, they are preferably such that, when the banaba extract is processed into banaba extract concentrate described later, the proportion of corosolic acid is 0.1-15 mg per 100 mg of the concentrate. Further, the proportion of corosolic acid is preferably 0.2-12 mg and more preferably 0.5-10 mg per 100 mg of the concentrate. Suitable extraction methods and conditions are as follows.

Method 1: Ethanol or an aqueous ethanol solution (50-80 wt% ethanol content) is added to dried pulverized banaba leaves (raw material) at 5-20 times by weight and preferably 8-10 times by weight with respect to the raw material, and the mixture is heated to reflux for extraction at a temperature from normal temperature to 90°C and preferably from about 50°C to 85°C, for a period from 30 minutes to 2 hours. The extraction is repeated 2 or 3 times.

Method 2: Methanol or an aqueous methanol solution (50-90 wt% methanol content) is added at 3-20 times by weight to dried pulverized banaba leaves, and the mixture is heated to reflux for extraction in the same manner as in Method 1. The extraction procedure is preferably carried out at a temperature from normal temperature to 65°C for a period from 30 minutes to 2 hours. The extraction procedure may be carried out once or repeated two or more times.

Method 3: Hot water is added at 3-20 times by weight to dried pulverized banaba leaves, and the mixture is heated to reflux for extraction at a temperature of 50-90°C and preferably 60-85°C, for a period from 30 minutes to 2 hours.

The above Methods 1 to 3 for preparing banaba extract may be combined as required. For example, Method 1 and Method 2 can be combined together. Of these methods, Methods 1 and 2 are preferred, but Method 1 is particularly preferred.

To facilitate handling, banaba extract is usually processed into banaba concentrate by concentration and drying. Regarding post-extraction concentration and drying, if the concentrate is stored at high temperature for a long time, the active components can deteriorate, so this is preferably performed in a relatively short time. For this purpose, it is advantageous to perform the concentration and drying under reduced pressure. The extract obtained as described above is filtered, and then concentrated under reduced pressure at a temperature of 60°C or less. The obtained solid is dried at a temperature of 50-70°C under reduced pressure (a higher reduced pressure than during the concentration). The dried solid is then crushed to obtain a powdered concentrate. Banaba extract concentrate is not limited to the powder form, and may be processed into tablets or granules. The banaba extract concentrate obtained by these methods contains corosolic acid, banaba polyphenols and other active components.

From the banaba extract or banaba extract concentrate obtained as described hereinabove, corosolic acid (containing 99% or more corosolic acid) can be obtained by removing components other than corosolic acid (other components) using an extraction technique known in the art (e.g., liquid chromatography such as HPLC).

When purifying corosolic acid from banaba extract, the following method may be used. Specifically, after suspending the banaba extract in water, it is distributed in ether or hexane to first remove low polarity components. The aqueous layer is then successively eluted with water, methanol and acetone using Diaion HP-20 column chromatography or the like. The methanol fraction containing corosolic acid is then subjected to separation and purification by silica gel column chromatography and high performance liquid chromatography so as to isolate the corosolic acid. Purification is easier if low polarity components are removed by ether or hexane and separation is performed using Diaion HP-20 column chromatography or the like (particularly if the extract amount is large), but this is not absolutely necessary, and the extract may be directly separated by silica gel column chromatography and then finally purified by high performance liquid chromatography.

The corosolic acid which is isolated and purified from the banaba extract or banaba extract concentrate may be used as it is, or alternatively, there may be used corosolic acid of high purity obtained by acylating (e.g., acetylating) the corosolic acid and then removing the acyl groups. Acetylation of the corosolic acid may be carried out, for example, by first dissolving the corosolic acid isolated and purified as described above in anhydrous pyridine, adding acetic anhydride and allowing the mixture to stand at room temperature for about 12 hours, and then adding ice water to the reaction mixture and performing extraction several times (about 3 times) with chloroform. Next, the chloroform layer may be dewatered with sodium sulfate, the sodium sulfate removed by filtration, and then the chloroform distilled off under reduced pressure and recrystallization performed from hexane to obtain acetylcorosolic acid. By removing the acyl groups from the acylated corosolic acid thus obtained, corosolic acid of extremely high purity (effectively 100%) can be obtained.

### (Manufacture of sweetener (functional sweetener))

The corosolic acid obtained as described above is mixed with a sucrase inhibitor (such as L-arabinose or 1-deoxynojirimycin) or a digestion and absorption inhibitor (blood glucose level depressor) (such as indigestible dextrin), and this is added to sucrose to obtain a sweetener (functional sweetener).

The mixing may be performed by crushing each of the above-mentioned components, but sucrose may be heated at normal pressure or under high pressure (approximately 180°C at normal pressure) to melt it, and the corosolic acid, a sucrase inhibitor (such as L-arabinose or 1-deoxynojirimycin) or a digestion and absorption inhibitor (blood glucose level depressor) (such as indigestible dextrin) dissolved therein.

An appropriate consumption of corosolic acid is 0.1 (mg/day/person), and supposing that the daily consumption of sucrose is about 50 g, the sweetener (functional sweetener) of the present invention may contain one part per million to one part in ten (but preferably one part in 50,000) of corosolic acid with respect to sucrose. The amount of a sucrase inhibitor (such as L-arabinose or 1-deoxynojirimycin) or a digestion and absorption inhibitor (blood glucose level depressor) (such as indigestible dextrin) is arbitrary.

### Industrial Applicability

The sweetener of the present invention can be used in the food manufacturing industry in general, such as in the manufacture of cakes or drinks. Also, it can be used as an ingredient of health supplements targeted at diabetic patients and people at risk of diabetes.

## Claims

1. A sweetener wherein corosolic acid, and at least one selected from the group consisting of a sucrase inhibitor and indigestible dextrin, are added to sucrose.

2. The sweetener according to claim 1, wherein said sucrase inhibitor is L-arabinose.

3. The sweetener according to claim 1, wherein said sucrase inhibitor is 1-deoxynojirimycin.
